(19) Europäisches Patentamt
European Patent Office
Office européen des brevets

(11) **EP 3 477 298 B1**

(12) **EUROPEAN PATENT SPECIFICATION**

(45) Date of publication and mention
of the grant of the patent:
**30.09.2020 Bulletin 2020/40**

(51) Int Cl.:
***G01N 33/49*** *(2006.01)*  ***G01N 21/64*** *(2006.01)*
***G01N 33/483*** *(2006.01)*  ***G01N 21/552*** *(2014.01)*

(21) Application number: **17841433.0**

(22) Date of filing: **09.08.2017**

(86) International application number:
**PCT/JP2017/028880**

(87) International publication number:
**WO 2018/034208 (22.02.2018 Gazette 2018/08)**

(54) **MEASUREMENT METHOD**

MESSVERFAHREN

PROCÉDÉ DE MESURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.08.2016 JP 2016160756**

(43) Date of publication of application:
**01.05.2019 Bulletin 2019/18**

(73) Proprietor: **Konica Minolta, Inc.
Tokyo 100-7015 (JP)**

(72) Inventors:
• **OTANI, Makiko**
**Chiyoda-ku, Tokyo, 100-7015 (JP)**
• **NODA, Tetsuya**
**Chiyoda-ku, Tokyo, 100-7015 (JP)**
• **NAGAI, Fumio**
**Chiyoda-ku, Tokyo, 100-7015 (JP)**

(74) Representative: **Burton, Nick et al
Urquhart-Dykes & Lord LLP
Arena Point
Merrion Way
Leeds LS2 8PA (GB)**

(56) References cited:
EP-A1- 2 963 418        WO-A1-2015/129615
WO-A1-2016/039149    JP-A- H11 295 305
JP-A- 2012 088 299      JP-A- 2013 036 959
US-A- 5 385 539

**Description**

Technical Field

The present invention relates to a measurement method of measuring a substance to be measured in a specimen containing blood.

Background Art

In clinical examinations, if it is possible to quantitatively detect a fine amount of substance to be measured in a specimen such as protein or DNA at high sensitivity, it becomes possible to rapidly grasp a condition of a patient and treat. For example, in a case of measuring an antigen (substance to be measured) in blood, whole blood collected from the patient or plasma or serum acquired by separating blood cell components from the whole blood may be used as the specimen. In addition, in order to grasp the condition of the patient, it is necessary to measure an amount (concentration) of the substance to be measured with respect to the plasma or serum. However, since a proportion of the plasma or serum to the whole blood varies from patient to patient, in a case where a measurement value indicating the amount of the substance to be measured is acquired by using the whole blood as the specimen, it is necessary to correct the measurement value according to the proportion of the plasma or serum to the whole blood. At that time, a hematocrit value may be used for correcting the measurement value. A method of determining the amount of the substance to be measured on the basis of the hematocrit value and the above-described measurement value is known (for example, refer to Patent Literature 1).

In a measurement method disclosed in Patent Literature 1, blood in a specimen is first hemolyzed by an oxidizing agent and a surfactant, and a specimen is diluted. Next, a measurement value indicating an amount of a substance to be measured in the specimen in a state in which blood is hemolyzed is acquired. Next, an amount of hemoglobin in the specimen in the state in which the blood is hemolyzed is measured and a hematocrit value of the specimen is acquired. Finally, the measurement value is corrected on the basis of the hematocrit value.

According to the above-described procedure, in the measurement method disclosed in Patent Literature 1, the amount of the substance to be measured in plasma or serum is measured.

WO 2015/129615 discloses measuring an intensity of reflected excitation light, a resonance angle of excitation light, an intensity of plasmon-scattered light, or an enhancement angle of excitation light, and using the acquired measurement to obtain a whole-blood hematocrit value of a blood-derived specimen on top of a metal film on a measurement chip. The whole-blood hematocrit value is used to convert a first signal value that indicates how much of an analyte the specimen contains to a second signal value that indicates how much of said analyte the liquid part of the specimen contains.

EP 2963418 discloses a specimen analysis device which prepares a first measurement sample from a urine specimen, a diluting solution having no hemolytic action, and a staining solution that stains membranes, supplies the first measurement sample to a flow cell and emits laser light thereon, thereby receiving fluorescence emitted from the first measurement sample, and obtains a fluorescence signal. The specimen analysis device prepares a second measurement sample from the urine specimen, a diluting solution having a hemolytic action, and a staining solution that stains nuclei, supplies the second measurement sample to the flow cell and emits laser light thereon, thereby receiving fluorescence emitted from the second measurement sample, and obtains a fluorescence signal. Particles not having nucleic acids, including red blood cells, are detected based on information on the fluorescence signal obtained from the first measurement sample, and cells having nucleic acids, including white blood cells, are detected based on information on the fluorescence signal obtained from the second measurement sample.

Citation List

Patent Literature

Patent Literature 1: JP 2013-036959 A

Summary of Invention

Technical Problem

In the measurement method disclosed in Patent Literature 1, in both a step of acquiring the hematocrit value and a step of acquiring the measurement value, the specimen in the state in which the blood is hemolyzed is used. The inventors of the present invention confirmed by experiments that it is sometimes impossible to accurately measure depending on measurement items when the substance to be measured is measured as for the specimen in the state in which the blood is hemolyzed. This is probably because protease (proteolytic enzyme) in a red blood cell flows out of the red blood cell by hemolysis of the blood and decomposes the substance to be measured. As described above, according to the measurement method disclosed in Patent Literature 1, there is case in which the amount of the substance to be measured in the specimen cannot be measured with a high degree of accuracy.

The present invention provides a measurement method capable of measuring the measurement value indicating the amount of the substance to be measured in the specimen and the hematocrit value with a high degree of accuracy and measuring the amount of the substance to be measured in the specimen containing blood with a high degree of accuracy.

Solution to Problem

In order to solve the above-described problem, a measurement method according to claim 1 is provided. Preferred embodiments are defined in the appended claims.

Advantageous Effects of Invention

According to the present invention, it is possible to measure the amount of the substance to be measured in the specimen containing blood with a high degree of accuracy.

Brief Description of Drawings

Fig. 1 is a flowchart illustrating an example of steps included in a measurement method according to one example of the present invention.
Fig. 2 is a flowchart illustrating steps in a measuring step of an optical blank value illustrated in Fig. 1.
Fig. 3 is a view illustrating an example of configurations of a measurement chip and an SPFS device.
Fig. 4A is a graph illustrating accuracy when determining an amount of a substance to be measured in plasma by an absorbance method, and Fig. 4B is a graph illustrating accuracy when determining the amount of the substance to be measured in plasma by an absorbance method.

Detailed description of preferred embodiments

The present invention is hereinafter described in detail with reference to the drawings. Here, as a representative example of a measurement method according to the present invention, a measurement method utilizing surface plasmon-field enhanced fluorescence spectroscopy (hereinafter abbreviated as "SPFS") is described.

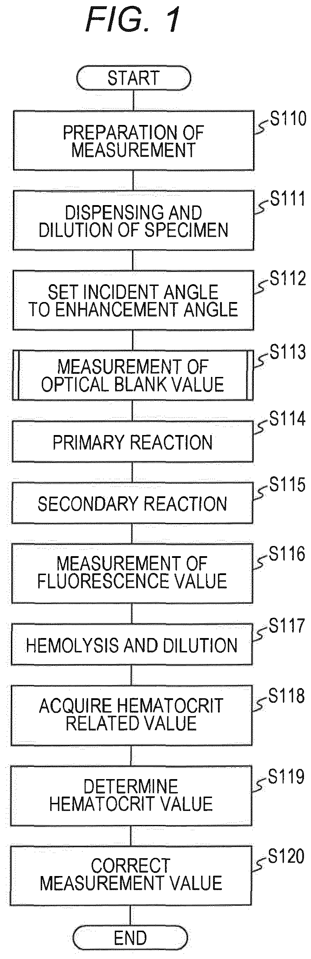

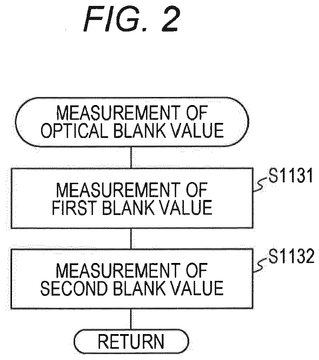

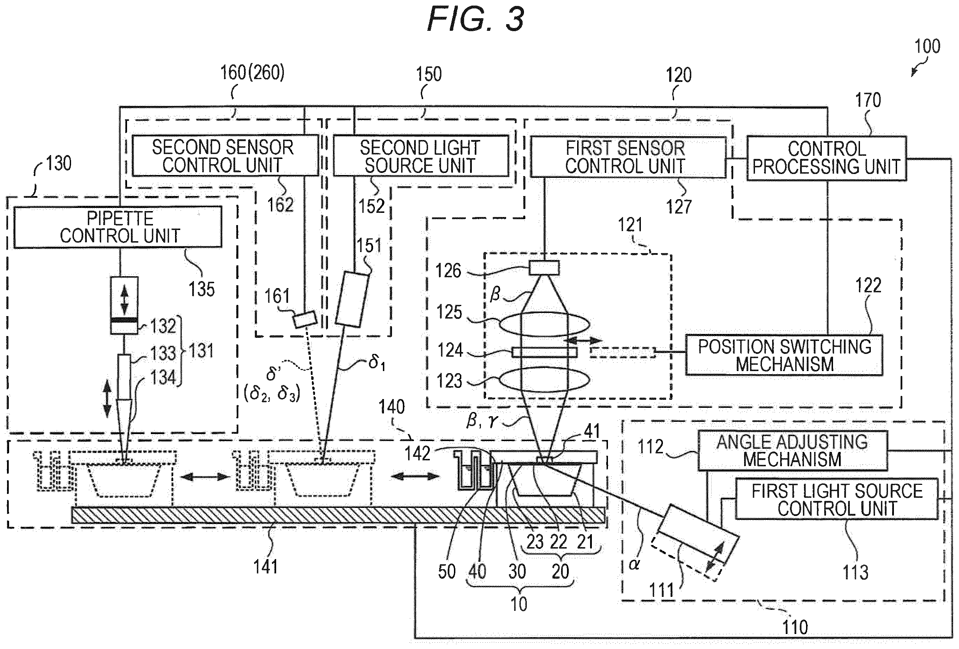

Fig. 1 is a flowchart illustrating an example of steps included in the measurement method according to one embodiment of the present invention. Fig. 2 is a flowchart illustrating steps in a measuring step of an optical blank value (step S113) illustrated in Fig. 1. Fig. 3 is a view illustrating an example of configurations of a measurement chip 10 and a measurement device (SPFS device) 100 which might be used for implementing the measurement method according to this embodiment. The measurement chip 10 and the SPFS device 100 are described later in detail.

The measurement method according to this embodiment is a measurement method in which an amount of a substance to be measured in a specimen containing blood is measured by using the measurement chip 10 including an accommodating unit (flow path 41 in this embodiment) for accommodating liquid. In this embodiment, as a measurement value indicating the amount of the substance to be measured, a fluorescence value which is a light amount of fluorescence β (signal) emitted from a fluorescent substance labeling the substance to be measured is measured.

The measurement method according to this example includes a step of preparing for measurement (step S110), a step of dispensing and diluting the specimen (step S111), a step of setting an incident angle to an enhancement angle (step S112), a step of measuring the optical blank value (step S113), a step of performing a primary reaction (step S114), a step of performing a secondary reaction (step S115), a step of measuring the fluorescence value (step S116), a step of performing hemolysis (step S117), a step of acquiring a hematocrit related value (step S118), a step of determining a hematocrit value (step S119), and a step of correcting the measurement value (step S120).

1) Preparation for Measurement

First, the measurement is prepared (step S110). Specifically, the measurement chip 10 is installed in a chip holder 142 arranged in an installation position of the SPFS device 100. Here, the "installation position" is a position for installing the measurement chip 10 in the SPFS device 100.

(Measurement Chip)

Here, the measurement chip 10 used in the SPFS device 100 is described. Fig. 3 is a view for illustrating an example of the configurations of the measurement chip 10 and the SPFS device 100. The measurement chip 10 includes a prism 20, a metal film 30, and a flow path lid 40. In this example, the flow path lid 40 of the measurement chip 10 is integrated with a liquid chip 50 for accommodating the liquid.

The prism 20 includes an incident surface 21, a film depositing surface 22, and an emission surface 23. The incident surface 21 allows excitation light $\alpha$ (referred to as "fourth light" in claims) from an excitation light emitting unit 110 to be described later to enter the prism 20. Here, the "excitation light" is light which directly or indirectly excites the fluorescent substance. The metal film 30 is arranged on the film depositing surface 22. The excitation light $\alpha$ entering the prism 20 is reflected by an interface (film depositing surface 22) between the prism 20 and the metal film 30 to be reflected light. The emission surface 23 emits the reflected light out of the prism 20.

A shape of the prism 20 is not especially limited. In this example, the shape of the prism 20 is a columnar body having a trapezoid as a bottom surface. A surface corresponding to one bottom side of the trapezoid is the film depositing surface 22, a surface corresponding to one leg is the incident surface 21, and a surface corresponding to the other leg is the emission surface 23. The trapezoid as the bottom surface is preferably an isosceles trapezoid. As a result, the incident surface 21 and the emission surface 23 become symmetrical, and an S wave component of the excitation light $\alpha$ is less likely to stay in the prism 20.

The incident surface 21 is formed such that the excitation light $\alpha$ from the excitation light emitting unit 110 is not reflected by the incident surface 21 to return to a light source of the SPFS device 100. In a case where a light source of the excitation light $\alpha$ is a laser diode (hereinafter also referred to as "LD"), when the excitation light $\alpha$ returns to the LD, an excited state of the LD is disturbed and a wavelength and an output of the excitation light $\alpha$ fluctuate. Therefore, in a scanning range centered on an ideal resonance angle or enhancement angle, an angle of the incident surface 21 is set so that the excitation light $\alpha$ does not enter the incident surface 21 perpendicularly.

Here, the "resonance angle" means the incident angle when a light amount of the reflected light of the excitation light $\alpha$ emitted from the emission surface 23 becomes the minimum in a case of scanning the incident angle of the excitation light $\alpha$ with respect to the metal film 30. In addition, the "enhancement angle" means the incident angle when a light amount of scattered light having the same wavelength as that of the excitation light $\alpha$ emitted above the measurement chip 10 (hereinafter referred to as "plasmon scattered light $\gamma$") becomes the maximum in a case where the incident angle of the excitation light $\alpha$ with respect to the metal film 30 is scanned. In this example, an angle between the incident surface 21 and the film depositing surface 22 and an angle between the film depositing surface 22 and the emission surface 23 are both approximately 80°.

Note that the resonance angle (and the enhancement angle in the close vicinity thereof) is roughly determined by a design of the measurement chip 10. Design elements are a refractive index of the prism 20, a refractive index of the metal film 30, a thickness of the metal film 30, an extinction coefficient of the metal film 30, the wavelength of the excitation light $\alpha$ and the like. The resonance angle and the enhancement angle are shifted by the substance to be measured captured on the metal film 30, but the amount is less than several degrees.

The prism 20 is made of a dielectric body transparent to the excitation light $\alpha$. The prism 20 has a birefringence characteristic more than little. Examples of materials of the prism 20 include resin and glass. Examples of the resin forming the prism 20 include polymethylmethacrylate (PMMA), polycarbonate (PC), and cycloolefin polymer. The material of the prism 20 is preferably resin having a refractive index of 1.4 to 1.6 and small birefringence.

The metal film 30 is arranged on the film depositing surface 22 of the prism 20. As a result, surface plasmon resonance (hereinafter abbreviated as "SPR") occurs between photons of the excitation light $\alpha$ incident on the film depositing surface 22 under a total reflection condition and free electrons in the metal film 30, and it is possible to generate localized field light (generally also referred to as "evanescent light" or "near field light") on a surface of the metal film 30. The localized field light reaches a distance approximately the wavelength of the excitation light $\alpha$ from the surface of the metal film 30. The metal film 30 may be formed on an entire surface on the film depositing surface 22 or on a part of the film depositing surface 22. In this example, the metal film 30 is formed on the entire surface of the film depositing surface 22.

In addition, in this example, the metal film 30 also serves as a reflecting unit which specularly reflects the light which passes through the flow path 41 so as to pass through the flow path 41 again. In this example, the metal film 30 specularly reflects first light $\delta_1$ including light of a wavelength absorbed by a red blood cell entering the measurement chip 10 at the flow path lid 40 toward the flow path lid 40.

On the metal film 30, a capturing body for capturing the substance to be measured is immobilized. On the metal film 30, a region in which the capturing body is immobilized is especially referred to as a "reaction field". The capturing body may be immobilized on an entire surface of the metal film 30 or may be immobilized on a part of the surface. From a viewpoint of suppressing the light which passes through the flow path 41 from being scattered by the capturing body, it is preferable that the capturing body is not immobilized in a region serving as the reflecting unit (a region where light specularly reflects) of the metal film 30. Also, the capturing body specifically binds to the substance to be measured. Therefore, the substance to be measured might be immobilized on the metal film 30 via the capturing body.

A type of the capturing body is not especially limited as long as this may capture the substance to be measured. For example, the capturing body is an antibody (primary antibody) capable of specifically binding to the substance to be measured, a fragment thereof, an enzyme capable of specifically binding to the substance to be measured or the like.

The material of the metal film 30 is not especially limited as long as this is metal capable of causing the surface plasmon resonance. Examples of the material of the metal film 30 include gold, silver, copper, aluminum, and alloys thereof. In this example, the metal film 30 is a gold thin film. Although the thickness of the metal film 30 is not especially limited, this is preferably in a range from 20 to 60 nm from a viewpoint of efficiently causing the SPR. A method of forming the metal film 30 is not especially limited. Examples of the method of forming the metal film 30 include sputtering, vapor deposition, and plating.

The flow path lid 40 is arranged on the metal film 30. In a case where the metal film 30 is formed only on a part of the film depositing surface 22 of the prism 20, the flow path lid 40 may also be arranged on the film depositing surface 22. In this example, the flow path lid 40 is arranged on the metal film 30. By arranging the flow path lid 40 on the metal film 30, the accommodating unit for accommodating liquid is formed on the metal film 30. In this example, the accommodating unit is the flow path 41 through which the liquid flows. The flow path 41 includes a bottom surface, a top surface, and a pair of side surfaces connecting the bottom surface and the top surface. In this specification, a surface of the flow path 41 on the prism 20 side is referred to as the "bottom surface of the flow path", and a surface of the flow path 41 opposed to the bottom surface of the flow path 41 is referred to as the "top surface of the flow path". Also, an interval between the bottom surface of the flow path 41 and the top surface of the flow path 41 is set as a height of the flow path 41. Since the height of the flow path 41 might be precisely managed, it is preferable that the accommodating unit is the flow path 41 from a viewpoint of precisely managing an optical path length of the flow path 41 related to absorption of the light by the specimen.

A recess (flow path groove) is formed on a rear surface of the flow path lid 40. The flow path lid 40 is arranged on the metal film 30 (and the prism 20), and an opening of the recess is closed by the metal film 30, so that the flow path 41 is formed. From a viewpoint of sufficiently securing a region where the localized field light reaches, it is preferable that the height of the flow path 41 (a depth of the flow path groove) is large to some extent. From a viewpoint of reducing an amount of impurities mixed in the flow path 41, the height of the flow path 41 (the depth of the flow path groove) is preferably small. From such a viewpoint, the height of the flow path 41 is preferably in a range from 0.05 to 0.15 mm. Both ends of the flow path 41 are connected to an injection port and a discharge port not illustrated formed on the flow path lid 40 so as to allow the inside and the outside of the flow path 41 to communicate with each other.

The flow path lid 40 is preferably formed of a material transparent to the light (fluorescence $\beta$ and plasmon scattered light $\gamma$) emitted from an upper side of the metal film 30 and the first light $\delta_1$ (and the light having the same wavelength as that of the first light $\delta_1$) including the light of the wavelength absorbed by the red blood cell emitted toward the metal film 30. The fact that the material of the flow path lid 40 is transparent to the first light $\delta_1$ is preferable from a viewpoint of suppressing scattering of light which becomes noise and measuring the hematocrit value with a high degree of accuracy. Examples of the material of the flow path lid 40 include glass and resin. Examples of the resin include polymethylmethacrylate resin (PMMA). Also, the other part of the flow path lid 40 may be formed of an opaque material as long as this is transparent to the above-described light. The flow path lid 40 is joined to the metal film 30 or the prism 20 by, for example, bonding with a double-sided tape, an adhesive and the like, laser welding, ultrasonic welding, crimping using a clamp member and the like.

The measurement chip 10 is usually exchanged for each measurement is made. Also, the measurement chip 10 is preferably a structure a length of each piece of which is several millimeters to several centimeters, but this may also be a smaller structure or a larger structure not included in a category of "chip".

Note that, in a case where a stored reagent is present on the metal film 30 of the measurement chip 10, the stored reagent is removed by washing the metal film 30 so that the capturing body may appropriately capture the substance to be measured.

2) Dispensing and Dilution of Specimen

Then, the specimen is dispensed and diluted (step S111). Specifically, the specimen is divided into a first specimen for measurement value (fluorescence value) measurement and a second specimen for hematocrit value measurement. At step S111, the first specimen is further diluted from a viewpoint of measurement accuracy and measurement sensitivity. Unless the first specimen is diluted, an amount of absorption (nonspecific adsorption) of impurities in the specimen to the capturing body increases and noise increases, and as a result, the measurement accuracy might be deteriorated. In addition, in a case where the amount of the substance to be measured is too large as compared with an amount of the capturing body, the amount of the substance to be measured which may be captured by the capturing body is saturated, and it becomes impossible to specify concentration of a highly concentrated specimen. As a diluent, for example, physiological saline may be used. The first specimen is diluted 1 to 500 times, for example.

It is preferable that the dispensing of the specimen is performed before a blood cell component in the specimen settles out. By dividing the specimen into the first specimen and the second specimen before the specimen is separated into a blood cell component and a supernatant component, a blood cell amount contained in the first specimen and a blood cell amount contained in the second specimen may be made equivalent to each other. From such a viewpoint, it is preferable that the dispensing of the specimen is performed after sufficiently mixing the specimen in a container in which blood is stored, for example, within a blood collecting tube. In addition, it is preferable that the specimen is dispensed in the SPFS device 100 before the primary reaction (step S114); for example, this is preferably performed within 10 minutes before the step of performing the primary reaction.

3) Set Incident Angle to Enhancement Angle

Next, the incident angle of the excitation light $\alpha$ with respect to the metal film 30 (film depositing surface 22) is set to the enhancement angle (step S112). Specifically, first, reference liquid transparent to the excitation light $\alpha$ is provided in the flow path 41. Next, in a state in which the reference liquid is present in the flow path 41, the excitation light $\alpha$ is applied to a rear surface of the metal film 30 corresponding to the region where the capturing body is immobilized via the prism 20 while scanning the incident angle of the excitation light $\alpha$ with respect to the metal film 30 and the plasmon scattered light $\gamma$ generated in the measurement chip 10 is detected. As a result, data including a relationship between the incident angle of the excitation light $\alpha$ and the light amount of the plasmon scattered light $\gamma$ is acquired. By analyzing the acquired data, the enhancement angle which is the incident angle at which the light amount of the plasmon scattered light $\gamma$ becomes the maximum might be determined. Finally, the incident angle of the excitation light $\alpha$ with respect to the metal film 30 (film depositing surface 22) is set to the determined enhancement angle.

Note that the enhancement angle is determined by the material and shape of the prism 20, the thickness of the metal film 30, the refractive index of the liquid in the flow path 41 and the like, but this slightly fluctuates by various factors such as the type and amount of the capturing body in the flow path 41 and an error in shape of the prism 20. Therefore, it is preferable to determine the enhancement angle each time the measurement is performed. The enhancement angle is determined on the order of approximately 0.1°.

4) Measurement of Optical Blank Value

Next, the optical blank value is measured (step S113). In this example, the optical blank value includes a first blank value used to determine the fluorescence value (measurement value) and a second blank value used to determine the hematocrit value. Here, the "first blank value" means the amount of light of the same wavelength as that of the fluorescence $\beta$ emitted above the measurement chip 10 in a state in which the reference liquid transparent to the first light $\delta_1$ is present in the flow path 41. Also, the "second blank value" means an amount of third light $\delta_3$ acquired when the first light $\delta_1$ passes through the reference liquid in the flow path 41 when the first light $\delta_1$ is applied to the metal film 30 in a state in which the reference liquid is present in the flow path 41.

In this example, the reference liquid is transparent to the excitation light $\alpha$ and the first light $\delta_1$. Note that a refractive index of the reference liquid is preferably the same as or equivalent to a refractive index of the specimen. As a result, it becomes possible to make reflectance of the first light $\delta_1$ on the bottom surface of the flow path groove (recess) of the flow path lid 40 (top surface of the flow path) the same or equivalent and to make reflectance of the light on the surface of the metal film 30 (reflecting unit) the same or equivalent between a case in which the reference liquid is present in the flow path 41 and a case in which the specimen is present in the flow path 41.

As illustrated in Fig. 2, at step S113, the first blank value is first measured (step S1131). At the same time as the metal film 30 (film depositing surface 22) is irradiated with the excitation light $\alpha$, the light amount of the light of substantially the same wavelength as the fluorescence $\beta$ is detected. As a result, it is possible to measure the light amount (first blank value) of light which becomes noise in the measurement of the fluorescence value (step S116).

Next, the second blank value is measured (step S1132). In a state in which the reference liquid is present in the flow path 41, the first light $\delta_1$ is applied to the surface of the metal film 30 corresponding to the region where the capturing body is immobilized via the flow path lid 40, and at the same time, the third light $\delta_3$ reflected by the metal film 30 is detected. As a result, it is possible to measure the light amount (second blank value) of light which becomes noise in the measurement of the hematocrit value. The second blank value may be absorbance $OD_1$ of the reference liquid represented by following equation (1).
[Equation 1]

$$OD_1 = -\log \frac{I_1}{I_0} \qquad \cdots \quad (1)$$

[In equation (1) above, $OD_1$ represents the absorbance of the reference liquid, $I_0$ represents the light amount of the first light $\delta_1$, and $I_1$ represents the light amount of the third light $\delta_3$.]

5) Primary Reaction

Next, the substance to be measured in the specimen and the capturing body on the metal film 30 are allowed to react (primary reaction; step S114). Specifically, the reference liquid in the flow path 41 is discharged and the first specimen diluted at step S111 is provided in the flow path 41. As a result, in a case where the substance to be measured is present in the specimen, at least a part of the substance to be measured may be captured by the capturing body on the metal film 30. Note that the first specimen is not hemolyzed. Thereafter, the interior of flow path 41 is washed with a buffer solution and the like to remove substances not captured by the capturing body. Note that examples of the substance to be measured include troponin, myoglobin, and creatine

6) Secondary Reaction

Subsequently, the substance to be measured captured by the capturing body on the metal film 30 is labeled with the fluorescent substance (secondary reaction; step S115). Specifically, a fluorescent labeling solution is provided in the flow path 41. As a result, the substance to be measured may be labeled with the fluorescent substance. The fluorescent labeling solution is, for example, a buffer solution containing an antibody (secondary antibody) labeled with the fluorescent substance. Thereafter, the interior of the flow path 41 is washed with the buffer solution and the like to remove free fluorescent substances and the like.

7) Measurement of Fluorescence Value

Next, the fluorescence emitted from the fluorescent substance labeling the substance to be measured in the reaction field is detected and the fluorescence value is measured (step S116). First, the buffer solution for measurement is provided in the flow path 41. In a state in which the substance to be measured contained in the first specimen is immobilized and the first specimen and the second specimen are not present, the excitation light $\alpha$ is applied to the rear surface of the metal film 30 corresponding to the region where the capturing body is immobilized via the prism 20 at the incident angle at which the surface plasmon resonance occurs. At the same time, the fluorescence $\beta$ (signal) generated in the measurement chip 10 is detected. As a result, it is possible to acquire the fluorescence value (measurement value) which is the light amount of the fluorescence $\beta$ which indicates the amount of the substance to be measured in the first specimen in a state in which blood is not hemolyzed. Note that, in this specification, "a state in which no specimen is present" means a state in which operation of removing the specimen from the flow path 41 is performed. That is, it suffices that there is substantially no specimen in the flow path 41, and a small amount of specimen which cannot be removed may be left in the flow path 41.

8) Hemolysis and Dilution

Next, blood in the second specimen dispensed at step S111 is hemolyzed and diluted (step S117). Specifically, a hemolytic agent is provided in the second specimen. As a result, the blood in the second specimen may be hemolyzed and diluted. At that time, the second specimen is diluted, for example, 1 to 20 times. At that time, in a case where a dilution ratio of the second specimen is one, this means that the second specimen is not diluted. The larger the dilution ratio, the smaller an amount of light absorbed by the specimen. Therefore, when the dilution ratio is too large, sufficient measurement resolution cannot be acquired.

In addition, a type of the hemolytic agent is not especially limited as long as this may hemolyze blood, and this may be appropriately selected from known hemolytic agents. The hemolytic agent is, for example, a surfactant. Examples of types of surfactant include an anionic surfactant, a cationic surfactant, and a nonionic surfactant. Among them, the surfactant is preferably the anionic surfactant.

9) Acquisition of Hematocrit Related Value

Next, the hematocrit related value is acquired (step S118). Specifically, the specimen in the state in which blood is hemolyzed is provided in the flow path 41. Next, in a state in which the second specimen in a state in which the blood is hemolyzed is present in the flow path 41, at the same time as the first light $\delta_1$ is applied to the metal film 30, second light $\delta_2$ acquired when this passes through the second specimen in the flow path 41, reflected by the metal film 30, and passes through again the second specimen in the flow path 41 is detected. As a result, the light amount of the second light $\delta_2$ may be measured. Note that the light amount of the second light $\delta_2$ may be absorbance $OD_2$ of the second specimen represented by following equation (2).

[Equation 2]

$$OD_2 = -\log\frac{I_2}{I_0} \qquad \cdots \ (2)$$

[In equation (2) above, $OD_2$ represents the absorbance of the second specimen, $I_0$ represents the light amount of the first light $\delta_1$, and $I_2$ represents the light amount of the second light $\delta_2$.]

Next, the hematocrit value determined on the basis of a detection result of the second light $\delta_2$ is corrected on the basis of a detection result of the third light $\delta_3$. The absorbance $OD_2$ of the second specimen includes a signal component resulting from the absorption of the light by the second specimen and a noise component (second blank value) caused by other factors. Therefore, by subtracting the noise component (second blank value) acquired at step S1132 from the absorbance $OD_2$ of the specimen acquired at step S118, the signal component may be calculated. On the basis of the measurement value (the light amount of the second light $\delta_2$ or the absorbance $OD_2$ of the specimen) acquired at step S118 and the measurement value acquired at the step S1132 (the light amount of the third light $\delta_3$ or the absorbance $OD_1$ of the reference liquid), a hematocrit related value Hct' represented by following equation (3) may be calculated.

[Equation 3]

$$Hct' = -\log\frac{I_2}{I_1} \qquad \cdots \ (3)$$

[In equation (3) above, Hct' represents the hematocrit related value, $I_1$ represents the light amount of the third light $\delta_3$, and $I_2$ represents the light amount of the second light $\delta_2$.]

Note that the absorbance of the specimen (hemoglobin) changes according to the wavelength of the first light $\delta_1$. It is preferable to adjust temperature of the light source of the first light $\delta_1$ to be kept constant from a viewpoint of stabilizing the wavelength of the first light $\delta_1$ and measuring the absorbance with a high degree of accuracy.

10) Determination of Hematocrit Value

Next, on the basis of the detection result of the second light $\delta_2$, the hematocrit value is determined (step S119). In this example, a hematocrit value Hct is calculated by multiplying the hematocrit related value acquired at step S118 by a predetermined correction coefficient.

Note that, as described above, the absorbance changes according to the wavelength of the first light $\delta_1$. Therefore, from a viewpoint of acquiring a more correct hematocrit value, it is preferable to correct the hematocrit value on the basis of the wavelength of the first light $\delta_1$. Consider, for example, a case where the hematocrit value is calculated with a reference value of the wavelength of the first light $\delta_1$ set to 520 nm. In this case, when the wavelength of the second light $\delta_2$ detected is 530 nm, the hematocrit value Hct may be corrected so as to be the value when the wavelength of the first light $\delta_1$ is 520 nm in consideration of a shift amount of an absorption rate (absorption amount) of the red blood cell corresponding to a shift amount (10 nm) between the measurement value and the reference value.

In addition, the hematocrit value may also be corrected on the basis of the height of the flow path 41 or the optical path length in the flow path 41 of the light which becomes the second light $\delta_2$. Consider, for example, a case where the hematocrit value is calculated with the reference value of the height of the flow path 41 set to 100 $\mu$m. In this case, when the measurement value of the height of the flow path 41 is 110 $\mu$m, the hematocrit value may be corrected so as to be the value when the height of the flow path 41 is 100 $\mu$m in consideration of a change amount of the absorption rate (absorption amount) of the red blood cell corresponding to the shift amount (10 $\mu$m) between the measurement

value and the reference value, that is, the shift amount of the height of the flow path 41.

11) Correction of Measurement Value

Finally, the measurement value (fluorescence value) is corrected on the basis of the hematocrit value (step S120). The fluorescence value includes the fluorescent component (signal component) derived from the fluorescent substance which labels the substance to be measured and the noise component (first blank value) caused by the factors other than the fluorescent substance. Therefore, by subtracting the first blank value acquired at step S1131 from the fluorescence value acquired at step S116, it is possible to calculate the measurement value (signal component) indicating the amount of the substance to be measured. Furthermore, by multiplying the calculated measurement value by a conversion coefficient c expressed by following equation (4), the calculated measurement value may be converted into the amount of the substance to be measured in plasma.

[Equation 4]

$$c = \frac{(df - 1) + (1 - Hct)}{df(1 - Hct)} \qquad \cdots \ (4)$$

[In equation (4) above, Hct represents the hematocrit value (0 to 100%) and df represents the dilution ratio of the diluent.]

By the above-described procedure, the amount (concentration) of the substance to be measured in the plasma may be determined.

Note that, in this example, a mode in which the step of detecting the second light $\delta_2$ (acquisition of the hematocrit related value; step S118) is performed after the step of acquiring the measurement value (measurement of the fluorescence value; step S116) is described. In a case where the detection of the second light $\delta_2$ and the detection of the fluorescence $\beta$ are performed in the same measurement chip 10 (flow path 41), it is preferable to perform the step of detecting the second light $\delta_2$ after the step of acquiring the measurement value from a viewpoint of acquiring measurement values with a high degree of accuracy. This is because it is possible to suppress the reaction between the substance to be measured in the second specimen to which the hemolytic agent is added and the capturing body on the metal 30 film by performing the step of detecting the second light $\delta_2$ after the step of acquiring the measurement value.

Also, in this example, a mode in which the step of hemolyzing the blood in the second specimen (step S117) is performed after the step of acquiring the measurement value (measurement of the fluorescence value; step S116) is described. In a case where a pipette handling the hemolytic agent and a pipette handling the specimen are the same, it is preferable that the step of hemolyzing the blood in the second specimen (step S117) is performed after the step of acquiring the measurement value (measurement of the fluorescence value; step S116) from a viewpoint of acquiring the measurement value with a high degree of accuracy. This is because, since the step of hemolyzing the blood in the second specimen is performed after the step of acquiring the measurement value, it is possible to prevent the hemolytic agent from remaining in the pipette and prevent the remaining hemolytic agent from mixing into the first specimen.

Also, in this example, a mode in which the step of dispensing the specimen (step S111) is performed before the step of the primary reaction (step S114) is described. The fact that the step of dispensing the specimen is performed within 10 minutes before the step (primary reaction step) of providing the first specimen in the flow path 41 is preferable from a viewpoint of determining the amount of the substance to be measured in plasma or serum with a high degree of accuracy. This is because, since the step of dispensing the specimen is performed within 10 minutes before the step of the primary reaction, it is possible to dispense the specimen before the blood cell component settles out, and make the hematocrit value of the first specimen and the hematocrit value of the second specimen the same or equivalent. As a result, it is possible to accurately correct the measurement value acquired using the first specimen on the basis of the hematocrit value acquired using the second specimen.

Also, from a viewpoint of measuring the hematocrit value with a high degree of accuracy, the measurement of the second blank value may be performed after the measurement of the fluorescence value (step S116). This may shorten a time interval between the measurement of the second blank value (step S1132) and the acquisition of the hematocrit related value (step S118), thereby making an effect of fluctuation in power of the light source or fluctuation in wavelength of the first light $\delta_1$ caused by the change in temperature of the light source. Furthermore, it is conceivable that a scattering state of the first light $\delta_1$ on the surface of the metal film 30 changes due to the primary reaction and the secondary reaction, but by measuring the second blank value (step S1132) and acquiring the hematocrit related value (step S118) after the first reaction and the second reaction, there is no effect of the change in the scattering state described above. From such a viewpoint, it is preferable to measure the second blank value (step S1132) after the measurement of the fluorescence value (step S116).

Also, in the above-described example, a mode in which the step of setting the incident angle to the enhancement angle (step S112), the step of measuring the optical blank value (step S113), and the step of performing the primary reaction (step S114) are performed in this order is described. However, in the measurement method according to the present invention, the order is not limited to this. For example, the incident angle may be set to the enhancement angle after the primary reaction is performed, or the primary reaction may be performed after measuring the optical blank value.

Also, in the description above, after the step of performing the primary reaction (step S114), the step of performing the secondary reaction (step S115) is performed (two-step method). However, a timing of labeling the substance to be measured with the fluorescent substance is not especially limited. For example, before introducing a specimen solution into the flow path 41 of the measurement chip 10, the labeling solution may be added to the specimen solution to label the substance to be measured with the fluorescent substance in advance. Alternatively, the specimen solution and the labeling solution may also be simultaneously injected into the flow path 41 of the measurement chip 10. In the former case, the substance to be measured labeled with the fluorescent substance is captured by the capturing body by injecting the specimen solution into the flow path 41 of the measurement chip 10. In the latter case, the substance to be measured is labeled with the fluorescent substance and the substance to be measured is captured by the capturing body. In either case, both the primary reaction and the secondary reaction may be completed by introducing the specimen solution into the flow path 41 of the measurement chip 10 (one-step method).

(SPFS Device)

Next, an example of the SPFS device which operates in accordance with the measurement method according to this example and may measure the substance to be measured in the specimen is described. Fig. 3 is a configuration diagram illustrating an example of the configuration of the SPFS device 100. The SPFS device 100 includes the excitation light emitting unit 110, a signal detecting unit 120, a liquid sending unit 130, a transporting unit 140, a light emitting unit 150, a light detecting unit 160, and a control processing unit (processing unit) 170. The excitation light emitting unit 110 and the signal detecting unit 120 form a measurement value acquiring unit for acquiring the measurement value indicating the amount of the substance to be measured in the specimen. The light emitting unit 150 and the light detecting unit 160 form a hematocrit value acquiring unit for acquiring the hematocrit value of the specimen.

In Fig. 3, the light emitting unit 150 and the light detecting unit 160 are arranged along a paper surface for the sake of convenience, but the light emitting unit 150 and the light detecting unit 160 are arranged in a direction perpendicular to the paper surface of Fig. 3. Note that, in Fig. 3, an optical axis of light $\delta'$ is indicated by a dotted line for illustrating that an optical axis of the first light $\delta_1$ and the optical axis of the light $\delta'$ are included within a plane perpendicular to the paper surface of Fig. 3.

The SPFS device 100 is used in a state in which the above-described measurement chip 10 is mounted on the chip holder (holder) 142 of the transporting unit 140. As described above, the measurement chip 10 includes the prism 20, the metal film 30, and the flow path lid 40. The flow path lid 40 of the measurement chip 10 is integrated with the liquid chip 50. The prism 20 includes an incident surface 21, a film depositing surface 22, and an emission surface 23. The flow path 41 is formed between the prism 20 and the flow path lid 40.

The excitation light emitting unit 110 emits the excitation light $\alpha$ (fourth light). When the fluorescence $\beta$ is detected, the excitation light emitting unit 110 emits a P wave to the metal film 30 toward the incident surface 21 such that the surface plasmon resonance occurs on the metal film 30. The excitation light $\alpha$ is light which generates the localized field light exciting the fluorescent substance on the surface of the metal film 30 when this is applied to the metal film 30 via the prism 20 at the angle at which the surface plasmon resonance occurs. The excitation light emitting unit 110 includes a first light source unit 111, an angle adjusting mechanism 112, and a first light source control unit 113.

The first light source unit 111 emits light collimated and having constant wavelength and light amount so that a shape of an irradiation spot on the rear surface of the metal film 30 is substantially circular. The first light source unit 111 includes, for example, a light source, a beam shaping optical system, an APC mechanism, and a temperature adjusting mechanism (none of them is illustrated).

A type of the light source is not especially limited, and is, for example, the laser diode (LD). Other examples of the light source include laser light sources such as light emitting diodes and mercury lamps. The wavelength of the excitation light $\alpha$ emitted from the light source is, for example, in a range of 400 nm to 1000 nm. In a case where the excitation light $\alpha$ emitted from the light source is not a beam, the excitation light $\alpha$ is converted into the beam by a lens, a mirror, a slit and the like. Also, in a case where the excitation light $\alpha$ emitted from the light source is not monochromatic light, the excitation light $\alpha$ is converted into the monochromatic light by a diffraction grating and the like. Furthermore, in a case where the excitation light $\alpha$ emitted from the light source is not linear polarization, the excitation light $\alpha$ is converted into linear polarization light by a polarizer and the like.

The beam shaping optical system includes, for example, a collimator, a band pass filter, a linear polarization filter, a half wavelength plate, a slit, a zoom means and the like. The beam shaping optical system may include all of them or a part of them.

The collimator collimates the excitation light $\alpha$ emitted from the light source.

The band pass filter converts the excitation light $\alpha$ emitted from the light source into narrow band light having only a central wavelength. This is because the excitation light $\alpha$ emitted from the light source has a slight wavelength distribution width.

The linear polarization filter makes the excitation light $\alpha$ emitted from the light source the linear polarization light.

The half wavelength plate adjusts a polarization direction of the light so that the P wave component is incident on the metal film 30.

The slit and the zoom means adjust a beam diameter, a contour shape and the like of the excitation light $\alpha$ emitted from the light source so that the shape of the irradiation spot on the rear surface of the metal film 30 becomes a circle of a predetermined size.

The APC mechanism controls the light source so that an output of the light source is constant. More specifically, the APC mechanism detects an amount of light branched from the excitation light $\alpha$ with a photodiode not illustrated and the like. Then, the APC mechanism controls input energy by a recurrent circuit, thereby controlling the output of the light source to be constant.

The temperature adjusting mechanism is, for example, a heater, a Peltier element and the like. The wavelength and energy of the excitation light $\alpha$ emitted from the light source might fluctuate depending on the temperature. Therefore, by keeping the temperature of the light source constant by the temperature adjusting mechanism, the wavelength and energy of the excitation light $\alpha$ emitted from the light source are controlled to be constant.

The angle adjusting mechanism 112 adjusts the incident angle of the excitation light $\alpha$ with respect to the metal film 30 (interface (film depositing surface 22) between the prism 20 and the metal film 30). In order to apply the light at a predetermined incident angle to a predetermined position of the metal film 30 via the prism 20, the angle adjusting mechanism 112 relatively rotates the optical axis of the excitation light $\alpha$ emitted from the light source and the chip holder 142. For example, the angle adjusting mechanism 112 rotates the first light source unit 111 around an axis orthogonal to the optical axis of the excitation light $\alpha$ on the metal film 30 (axis perpendicular to the paper surface of Fig. 3). At that time, a position of a rotational axis is set such that a position of the irradiation spot on the metal film 30 scarcely changes even if the incident angle is scanned. Especially, displacement of the irradiation position may be minimized by setting a position of a rotational center in the vicinity of an intersection (between the irradiation position on the film depositing surface 22 and the incident surface 21) of the optical axes of the excitation light $\alpha$ emitted from two light sources at both ends of a scanning range of the incident angle.

As described above, the angle at which the light amount of the plasmon scattered light $\gamma$ becomes maximum out of the incident angle of the excitation light $\alpha$ emitted from the light source to the metal film 30 is the enhancement angle. By setting the incident angle of the excitation light $\alpha$ emitted from the light source to the enhancement angle or the angle in the vicinity thereof, high-intensity fluorescence $\beta$ and plasmon scattered light $\gamma$ may be detected. Although a basic incident condition of the excitation light $\alpha$ emitted from the light source is determined by the material and shape of the prism 20, the thickness of the metal film 30, the refractive index of the liquid in the flow path 41 and the like, an optimal incident condition slightly fluctuates depending on the type and amount of the capturing body in the flow path 41, the error in shape of the prism 20 and the like. Therefore, it is preferable to acquire an optimum enhancement angle for each measurement.

The first light source control unit 113 controls various devices included in the first light source unit 111 to control the emission of the excitation light $\alpha$ from the first light source unit 111. The first light source control unit 113 is formed of, for example, a well-known computer or microcomputer including an arithmetic device, a control device, a storage device, an input device, and an output device.

The signal detecting unit 120 detects a signal (for example, fluorescence $\beta$, reflected light, or plasmon scattered light $\gamma$) generated in the measurement chip 10 when the excitation light emitting unit 110 applies the excitation light $\alpha$ to the metal film 30 at the incident angle at which the surface plasmon resonance occurs via the prism 20 in a state in which the substance to be measured in the specimen is present on the metal film 30. The substance to be measured may be immobilized in the flow path 41 or not. In this example, the signal detecting unit 120 detects the above-described signal in a state in which the substance to be measured contained in the specimen is immobilized on the metal film 30 and the specimen is not present in the flow path 41. The signal detecting unit 120 outputs a signal indicating a detected signal amount (for example, the light amount of the fluorescence $\beta$, the light amount of the reflected light $\delta'$ or the light amount of the plasmon scattered light $\gamma$) to the control processing unit 170. The signal detecting unit 120 includes a light receiving optical system unit 121, a position switching mechanism 122, and a first sensor control unit 127.

The light receiving optical system unit 121 is arranged on a normal to the metal film 30 of the measurement chip 10. The light receiving optical system unit 121 includes a first lens 123, an optical filter 124, a second lens 125, and a first light receiving sensor 126.

The position switching mechanism 122 switches a position of the optical filter 124 on the optical path or out of the optical path in the light receiving optical system unit 121. Specifically, when the first light receiving sensor 126 detects the fluorescence $\beta$, the optical filter 124 is arranged on the optical path of the light receiving optical system unit 121, and when the first light receiving sensor 126 detects the plasmon scattered light $\gamma$, the optical filter 124 is arranged outside the optical path of the light receiving optical system unit 121.

The first lens 123 is, for example, a condensing lens, and condenses light (signal) emitted from the upper side of the metal film 30. The second lens 125 is, for example, an image forming lens, and forms an image of the light condensed by the first lens 123 on a light receiving surface of the first light receiving sensor 126. Between the two lenses, the light is a substantially parallel light flux.

The optical filter 124 is arranged between the first lens 123 and the second lens 125. When detecting fluorescence, the optical filter 124 transmits only the fluorescent component out of the light incident on the optical filter 124 and removes an excitation light component (plasmon scattered light $\gamma$). As a result, it is possible to guide only the fluorescent component to the first light receiving sensor 126 and detect the fluorescence $\beta$ with a high S/N ratio. Examples of types of the optical filter 124 include an excitation light reflecting filter, a short wavelength cutting filter, and a band pass filter. Examples of the optical filter 124 include a filter including a multilayer film which reflects a predetermined light component and a color glass filter which absorbs a predetermined light component.

The first light receiving sensor 126 detects the fluorescence $\beta$ and the plasmon scattered light $\gamma$. The first light receiving sensor 126 has high sensitivity capable of detecting weak fluorescence $\beta$ from a minute amount of substance to be measured. The first light receiving sensor 126 is, for example, a photomultiplier tube (PMT), an avalanche photodiode (APD), a silicon photodiode (SiPD) and the like.

The first sensor control unit 127 controls detection of an output value of the first light receiving sensor 126, management of sensitivity of the first light receiving sensor 126 by the output value, change in the sensitivity of the first light receiving sensor 126 for acquiring an appropriate output value and the like. The first sensor control unit 127 is formed of, for example, a well-known computer or microcomputer including an arithmetic device, a control device, a storage device, an input device, and an output device.

The liquid sending unit 130 supplies the liquid in the liquid chip 50 into the flow path 41 of the measurement chip 10 held by the chip holder 142. Also, the liquid sending unit 130 removes liquid from the flow path 41 of the measurement chip 10. Furthermore, the liquid sending unit 130 dispenses and dilutes the liquid in the liquid chip 50. The liquid sending unit 130 includes a pipette 131 and a pipette control unit 135.

The pipette 131 includes a syringe pump 132, a nozzle unit 133 connected to the syringe pump 132, and a pipette chip 134 attached to a tip end of the nozzle unit 133. Reciprocating motion of a plunger in the syringe pump 132 quantitatively sucks and discharges the liquid in the pipette chip 134.

The pipette control unit 135 includes a driving device of the syringe pump 132 and a moving device of the nozzle unit 133. The driving device of the syringe pump 132 is a device for reciprocating the plunger of the syringe pump 132 and includes, for example, a stepping motor. For example, the moving device of the nozzle unit 133 freely moves the nozzle unit 133 in a vertical direction. The moving device of the nozzle unit 133 is formed of, for example, a robot arm, a two-axis stage, or a vertically movable turntable.

The pipette control unit 135 drives the syringe pump 132 to suck various types of liquid from the liquid chip 50 into the pipette chip 134. Then, the pipette control unit 135 moves the nozzle unit 133 to insert the pipette chip 134 into the flow path 41 of the measurement chip 10, and drives the syringe pump 132 to inject the liquid in the pipette chip 134 into the flow path 41. Also, after introducing the liquid, the pipette control unit 135 drives the syringe pump 132 to suck the liquid in the flow path 41 into the pipette chip 134. By sequentially exchanging the liquid in the flow path 41 in this manner, the capturing body and the substance to be measured are allowed to react in the reaction field (primary reaction) and the substance to be measured and the capturing body labeled with the fluorescent substance are allowed to react (secondary reaction). Also, the liquid sending unit 130 sucks or discharges the liquid in the liquid chip 50 in the above-described manner, thereby dispensing or diluting the specimen.

The transporting unit 140 transports the measurement chip 10 to fix. The transporting unit 140 includes a transporting stage 141 and the chip holder 142.

The transporting stage 141 moves the chip holder 142 in one direction and in the opposite direction. The transporting stage 141 also has a shape which does not interfere with the optical paths of the light such as the excitation light $\alpha$, the reflected light of the excitation light $\alpha$, the fluorescence $\beta$, the plasmon scattered light $\gamma$, the first light $\delta_1$, and the reflected light $\delta'$ of the first light $\delta_1$ (second light $\delta_2$ and third light $\delta_3$). The transporting stage 141 is driven by, for example, a stepping motor and the like.

The chip holder 142 is fixed to the transporting stage 141 and detachably holds the measurement chip 10. The chip holder 142 has a shape capable of holding the measurement chip 10 which does not interfere with the optical paths of the light such as the excitation light $\alpha$, the reflected light of the excitation light $\alpha$, the fluorescence $\beta$, the plasmon scattered light $\gamma$, the first light $\delta_1$, and the reflected light $\delta'$ of the first light $\delta_1$. For example, the chip holder 142 is provided with an opening through which the above-described light passes.

The light emitting unit 150 emits the first light $\delta_1$ including light of a wavelength absorbed by the red blood cell. In this example, the light emitting unit 150 emits the first light $\delta_1$ from the flow path 41 side toward the metal film 30. It is preferable that the first light $\delta_1$ contains light of a wavelength absorbed by hemoglobin contained in the red blood cell.

The light emitting unit 150 includes a second light source unit 151 and a second light source control unit 152.

The second light source unit 151 emits the first light $\delta_1$ which is collimated and has constant wavelength and light amount toward the metal film 30. The second light source unit 151 includes, for example, a light source, a collimator, an APC mechanism, and a temperature adjusting mechanism (none of them is illustrated). The collimator, the APC mechanism, and the temperature adjusting mechanism are similar to the collimator, the APC mechanism, and the temperature adjusting mechanism of the first light source unit 111, so that the description thereof is omitted. From a viewpoint of suppressing variation in wavelength of the first light $\delta_1$ due to temperature change of the light source, the second light source unit 151 preferably includes the temperature adjusting mechanism.

From a viewpoint of applying the first light $\delta_1$ to an inside of an outer edge of the metal film 30 and suppressing reduction in energy efficiency due to irradiation of the first light $\delta_1$ in a region other than the metal film 30, the light source is preferably the laser light source. The laser light source may irradiate the metal film 30 with the first light $\delta_1$ in a smaller irradiation spot as compared with that of a light source having low directivity such as an LED. The light source is, for example, a laser diode (LD). Other examples of the light source include laser light sources such as light emitting diodes and mercury lamps. A central wavelength of the first light $\delta_1$ from the light emitting unit 150 is, for example, 500 to 650 nm. In a case where the first light $\delta_1$ emitted from the light source is not a beam, the first light $\delta_1$ is converted into a beam by a lens, a mirror, a slit and the like.

The second light source control unit 152 controls various devices included in the second light source unit 151 and controls emission of the first light $\delta_1$ from the second light source unit 151. The second light source control unit 152 is formed of, for example, a well-known computer or microcomputer including an arithmetic device, a control device, a storage device, an input device, and an output device.

The light detecting unit 160 detects the light $\delta'$ acquired by the reflection of the first light $\delta_1$ in the measurement chip 10. For example, in a state in which the specimen is present in the flow path 41, the light detecting unit 160 detects the second light $\delta_2$ acquired when the first light $\delta_1$ passes through the specimen in the flow path 41, reflected by the metal film 30, and passes through again the specimen in the flow path 41 when the light emitting unit 150 emits the first light $\delta_1$ toward the metal film 30. The light detecting unit 160 outputs a signal indicating a light amount of the detected light $\delta'$. The light detecting unit 160 includes a second light receiving sensor 161 and a second sensor control unit 162.

The second light receiving sensor 161 detects the light $\delta'$ which is the first light $\delta_1$ reflected within the measurement chip 10. The second light receiving sensor 161 is, for example, a photomultiplier tube (PMT), an avalanche photodiode (APD), a silicon photodiode (SiPD) and the like.

The second sensor control unit 162 controls detection of an output value of the second light receiving sensor 161, management of sensitivity of the second light receiving sensor 161 by the output value, change in the sensitivity of the second light receiving sensor 161 for acquiring an appropriate output value and the like. The second sensor control unit 162 is formed of, for example, a well-known computer or microcomputer including an arithmetic device, a control device, a storage device, an input device, and an output device.

The light emitting unit 150 (second light source unit 151) and the light detecting unit 160 (second light receiving sensor 161) are preferably arranged such that a plane including the optical axis of the first light $\delta_1$ and the optical axis of the reflected light $\delta'$ of the first light $\delta_1$ is in a longitudinal direction of the flow path 41. As a result, even if the position of the irradiation spot of the first light $\delta_1$ is displaced in the longitudinal direction of the flow path 41, it is possible to suppress the irradiation of the region other than the flow path 41 with the first light $\delta_1$. Also, as compared with a case where the first light $\delta_1$ is perpendicularly incident on the metal film 30, in a case where the first light $\delta_1$ is obliquely incident on the metal film 30, the shape of the irradiation spot of the first light $\delta_1$ on the metal film 30 extends in one direction. The direction in which the irradiation spot extends depending on the incident angle of the first light $\delta_1$ with respect to the metal film 30 is preferably in the longitudinal direction of the flow path 41. As a result, as compared with a case where the direction in which the irradiation spot extends is in a lateral direction of the flow path 41, even if the position of the irradiation spot is displaced, the first light $\delta_1$ may be suppressed from being applied to the region other than the flow path 41 (metal film 30). Note that a cause of the positional displacement of the irradiation spot includes a positioning error of the chip holder 142 on the transporting stage 141, an installation error of the measurement chip 10 with respect to the chip holder 142 and the like.

The control processing unit 170 controls the angle adjusting mechanism 112, the first light source control unit 113, the position switching mechanism 122, the first sensor control unit 127, the pipette control unit 135, the transporting stage 141, the second light source control unit 152, and the second sensor control unit 162. The control processing unit 170 also serves as a processing unit which processes detection results of the signal detecting unit 120 (first light receiving sensor 126) and the light detecting unit 160 (second light receiving sensor 161). In this example, the control processing unit 170 determines the measurement value indicating the amount of the substance to be measured in the specimen on the basis of the detection result of the fluorescence $\beta$ by the signal detecting unit 120. In addition, the control processing unit 170 determines the hematocrit value of the specimen on the basis of the detection result of the second light $\delta_2$ by the light detecting unit 160. Along with this, the control processing unit 170 corrects the above-described measurement value on the basis of the hematocrit value. As a result, the control processing unit 170 determines the amount (concentration) of the substance to be measured in plasma or serum. In addition, predetermined information (for example, various conversion coefficients and data regarding calibration curve) and the like used when processing the above-described detection results may also be recorded in the control processing unit 170 in advance. In this example, a coefficient for converting the hematocrit related value to the hematocrit value is recorded in the control processing unit 170 in advance. The control processing unit 170 is formed of, for example, a well-known computer or microcomputer including an arithmetic device, a control device, a storage device, an input device, and an output device.

(Optical Path in SPFS Device)

As illustrated in Fig. 3, the excitation light $\alpha$ enters the prism 20 from the incident surface 21. The excitation light $\alpha$ entering the prism 20 is incident on the metal film 30 at a total reflection angle (angle at which SPR occurs). In this manner, the localized field light may be generated on the metal film 30 by irradiating the metal film 30 with the excitation light $\alpha$ at an angle at which the SPR occurs. By this localized field light, the fluorescent substance which labels the substance to be measured present on the metal film 30 is excited and the fluorescence $\beta$ is released. The SPFS device 100 detects the light amount (intensity) of the fluorescence $\beta$ emitted from the fluorescent substance. Note that, although not especially illustrated, the reflected light of the excitation light $\alpha$ on the metal film 30 is emitted out of the prism 20 from the emission surface 23.

Also, as illustrated in Fig. 3, in this example, the first light $\delta_1$ enters the flow path 41 (measurement chip 10) via the flow path lid 40. The first light $\delta_1$ is reflected within the measurement chip 10. The light reflected in the measurement chip 10 is emitted out of the flow path 41 (measurement chip 10) via the flow path lid 40. The SPFS device 100 detects the light emitted from the measurement chip 10.

(Effect)

The measurement value (fluorescence value) indicating the amount of the substance to be measured is acquired by the first specimen in a state in which the blood is not hemolyzed, and the hematocrit related value is acquired by the specimen of the second specimen in a state in which the blood is hemolyzed to determine the hematocrit value. Since the blood in the first specimen is not hemolyzed, it is possible to suppress protease (proteolytic enzyme) in the red blood cell from flowing out of the red blood cell to decompose the substance to be measured. As a result, the measurement value may be acquired with a high degree of accuracy. In addition, since the blood in the second specimen is hemolyzed, the effect of scattering of light by the red blood cell may be reduced. As a result, the hematocrit related value may be acquired with a high degree of accuracy, and the hematocrit value may be determined with a high degree of accuracy. Therefore, in the measurement method according to the invention, the measurement value indicating the amount of the substance to be measured in the specimen and the hematocrit value may be measured with a high degree of accuracy and the amount of the substance to be measured in the specimen containing blood may be measured with a high degree of accuracy.

Note that, in the measurement method according to this example, the light which becomes the second light $\delta_2$ is allowed to reciprocate in the flow path 41 by the metal film 30. This makes it possible to lengthen the optical path length of the light which becomes the second light $\delta_2$ in the flow path 41 as compared with a case where reflection by the metal film 30 is not utilized. As a result, it is possible to measure the hematocrit value with a high degree of accuracy by increasing the amount of light absorbed by the specimen. As a result, the amount of the substance to be measured may be determined with a high degree of accuracy.

Also, in the measurement method according to this example, it is possible to measure the second blank value at step S1132 and remove the effect of the noise component (step S118). Therefore, it is possible to measure the hematocrit value with a higher degree of accuracy.

Also, in this example, the second blank value is measured (step S1132) before the primary reaction (step S114). Therefore, even if the washing in the flow path 41 after the primary reaction is not sufficient and the blood remains in the flow path 41, the measurement of the second blank value is not affected by residual blood. Therefore, the hematocrit value may be measured with a high degree of accuracy. From such a point of view, it is preferable to measure the second blank value (step S1132) before the first reaction (step S114).

Furthermore, although a mode in which the SPFS method is used and the fluorescence value of the fluorescence $\beta$ from the fluorescent substance is measured as the measurement value is described in this example, the present invention is not limited to this mode. For example, it is also possible to measure the light amount of the reflected light of the excitation light $\alpha$ as the measurement value by utilizing an SPR method. Also, in the present invention, the measurement value may also be acquired by using an ELISA method, an RIfS method, a QCM method and the like.

[Reference Experiment]

In a reference experiment, a hematocrit value was measured by using an absorbance method, a microhematocrit method, or an electric resistance method, a measurement value indicating an amount of a substance to be measured previously acquired was corrected with the hematocrit value Hct, and results of the measurement were compared.

1. Acquisition of Measurement Value

As specimens, 24 types of whole blood containing cardiac troponin (cTn)I to which heparin was added were used. When acquiring the measurement value, blood in the specimen is not hemolyzed. Concentration of cTnI in the specimen was measured using a high-sensitivity automatic immunoassay device (SPFS device according to the above-described example) having a function of measuring the hematocrit value by the absorbance method.

2. Micro Hematocrit Method

The specimen in a state in which blood is not hemolyzed was placed in a capillary made of glass to be centrifuged for five minutes at 12000 rpm by using a well-known hematocrit centrifuge (Centec 3220; manufactured by KUBOTA CORPORATION, "Centec" is the registered trademark of the company). Using a measuring instrument attached to the hematocrit centrifuge, the hematocrit value of the specimen after the centrifugation was measured. Subsequently, on the basis of the measured hematocrit value, the concentration of cTnI in the specimen was corrected to the concentration of cTnI in plasma.

3. Absorbance Method

First, blood was hemolyzed with a surfactant. Next, the hematocrit value of the specimen in a state in which blood was hemolyzed was measured by an absorbance method using a high-sensitivity automatic immunoassay device. Subsequently, on the basis of the measured hematocrit value, the concentration of cTnI in the specimen was corrected to the concentration of cTnI in plasma.

4. Electric Resistance Method

Using a well-known hematocrit analyzer (i-STAT; manufactured by Abbott Point of Care Inc., "i-STAT" is the registered trademark of the company), the hematocrit value of the specimen in a state in which blood is not hemolyzed was measured by an electric resistance method. Subsequently, on the basis of the measured hematocrit value, the concentration of cTnI in the specimen was corrected to the concentration of cTnI in plasma.

A specimen number, cTnI concentration WB-cTnI in the specimen, the hematocrit value Hct and the cTnI concentration in the plasma (correction value A (reference value)) acquired by the microhematocrit method, the hematocrit value Hct, the cTnI concentration in plasma (correction value B), and a shift amount BiasB of the correction value B with respect to the reference value acquired by the absorbance method, and the hematocrit value Hct, the cTnI concentration (correction value C) in the plasma, and a shift amount BiasC of the correction value C with respect to the reference value acquired by the electric resistance method are illustrated in Table 1. In Table 1, BiasB is a value calculated by following equation (5) and BiasC is a value calculated by following equation (6).

[Equation 5]

$$BiasB = \frac{B - A}{(A + B)/2} \times 100 \qquad \cdots (5)$$

[Equation 6]

$$BiasC = \frac{C - A}{(A + C)/2} \times 100 \qquad \cdots (6)$$

[Table 1]

| SPECIMEN NUMBER | WB-cTnI [pg/mL] | MICROHEMATOCRIT METHOD | | ABSORBANCE METHOD | | | ELECTRIC RESISTANCE METHOD | | |
|---|---|---|---|---|---|---|---|---|---|
| | | Hct [%] | CORRECTION VALUE A [pg/mL] | Hct [%] | CORRECTION VALUE B [pg/mL] | Bias B [%] | Hct [%] | CORRECTION VALUE C [ pg/mL] | Bias C [%] |
| 1 | 50.0 | 37.0 | 69.6 | 39.8 | 72.1 | +3.52 | 39.0 | 71.3 | +2.5 |
| 2 | 55.2 | 30.0 | 71.0 | 29.4 | 70.5 | -0.60 | 34.0 | 74.2 | +4.4 |
| 3 | 60.4 | 39.0 | 86.1 | 36.1 | 83.1 | -3.57 | 40.0 | 87.2 | +1.3 |
| 4 | 61.1 | 40.0 | 88.3 | 38.9 | 87.1 | -1.34 | 40.0 | 88.3 | 0 |
| 5 | 61.8 | 38.0 | 87.1 | 38.4 | 87.4 | +0.46 | 39.0 | 88.1 | +1.2 |
| 6 | 74.5 | 30.0 | 95.8 | 28.0 | 93.8 | -2.07 | 32.0 | 97.9 | +2.2 |
| 7 | 76.0 | 36.0 | 104.5 | 38.4 | 107.6 | +2.91 | 34.0 | 102.1 | -2.3 |
| 8 | 90.4 | 39.0 | 128.9 | 40.6 | 131.6 | +2.06 | 38.0 | 127.3 | -1.2 |
| 9 | 95.1 | 39.0 | 135.6 | 35.8 | 130.5 | -3.88 | 37.0 | 132.3 | -2.5 |
| 10 | 123.8 | 45.0 | 191.3 | 47.1 | 197.1 | +2.99 | 56.0 | 228.8 | +17.9 |
| 11 | 181.3 | 37.0 | 252.3 | 39.8 | 261.1 | +3.45 | 38.0 | 255.4 | +1.2 |
| 12 | 198.0 | 46.0 | 310.4 | 46.2 | 311.4 | +0.32 | 51.0 | 335.4 | +7.7 |
| 13 | 198.1 | 43.5 | 299.8 | 44.3 | 303.2 | +1.13 | 49.0 | 325.0 | +8.1 |
| 14 | 203.2 | 41.0 | 297.3 | 41.4 | 298.9 | +0.52 | 44.0 | 309.6 | +4.1 |
| 15 | 257.1 | 46.5 | 406.1 | 49.0 | 421.5 | +3.74 | 50.0 | 428.5 | +5.4 |
| 16 | 593.0 | 46.0 | 929.8 | 44.0 | 904.1 | -2.80 | 47.0 | 943.6 | +1.5 |
| 17 | 598.8 | 37.0 | 833.3 | 37.4 | 837.1 | +0.46 | 37.0 | 833.3 | 0 |
| 18 | 662.1 | 31.0 | 860.4 | 28.9 | 841.3 | -2.25 | 30.0 | 851.2 | -1.1 |
| 19 | 748.4 | 50.0 | 1247.3 | 49.3 | 1233.5 | -1.11 | 49.0 | 1227.7 | -1.6 |
| 20 | 752.6 | 38.0 | 1060.1 | 37.8 | 1057.5 | -0.25 | 37.0 | 1047.3 | -1.2 |
| 21 | 785.9 | 43.0 | 1181.2 | 41.9 | 1164.4 | -1.43 | 43.0 | 1181.2 | 0 |
| 22 | 814.9 | 35.0 | 1107.4 | 33.1 | 1083.3 | -2.20 | 34.0 | 1094.8 | -1.2 |
| 23 | 908.4 | 43.0 | 1365.3 | 42.3 | 1351.7 | -1.00 | 41.0 | 1329.3 | -2.7 |
| 24 | 954.6 | 39.0 | 1361.5 | 37.3 | 1333.2 | -2.10 | 38.0 | 1344.7 | -1.2 |

EP 3 477 298 B1

16

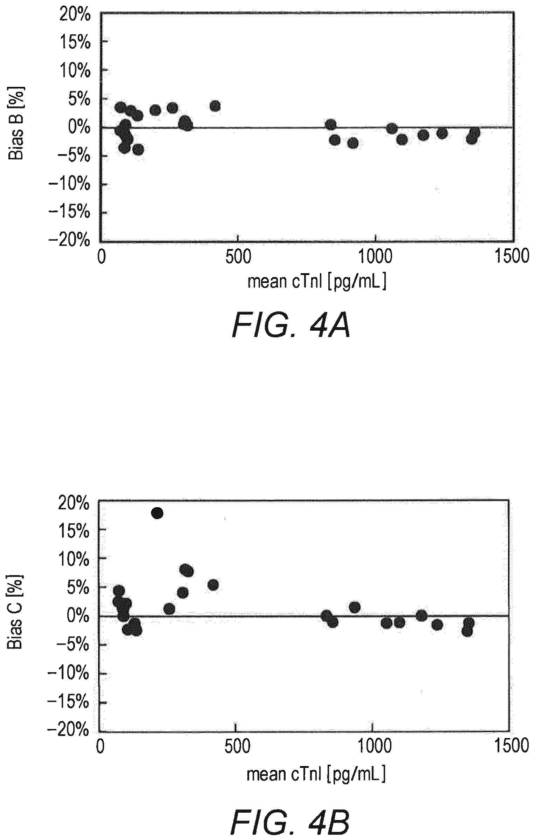

Fig. 4A is a graph illustrating accuracy of the amount of the substance to be measured in the plasma determined by the absorbance method, and Fig. 4B is a graph illustrating the accuracy of the amount of the substance to be measured in the plasma determined by the electric resistance method. In Fig. 4A, mean cTnI concentration is plotted along the abscissa and BiasB which is the shift amount of the correction value B with respect to the correction value A (reference value) is plotted along the ordinate. In Fig. 4B, mean cTnI concentration is plotted along the abscissa and BiasC which is the shift amount of the correction value C with respect to the correction value A (reference value) is plotted along the ordinate.

As illustrated in Fig. 4A and Table 1, BiasB was within 5% in any of the specimens used in this embodiment. On the other hand, as illustrated in Fig. 4B and Table 1, BiasC was more than 5% in at least a part of the specimens used in this embodiment, and it is confirmed that BiasC tends to be larger in the specimen having a high hematocrit value of equal to or more than 50%. In this manner, it is understood that the absorbance method using the specimen containing hemolyzed blood may determine the amount of the substance to be measured in the plasma with a higher degree of accuracy as compared with the electric resistance method using the specimen containing blood which is not hemolyzed. In addition, compared to the micro hematocrit method, with the absorbance method, it is unnecessary to separately prepare a device such as a centrifuge, and it is possible to simply and highly accurately measure in a device for measuring the substance to be measured. As described above, according to the present invention, it is not necessary to separately prepare a device for measuring the hematocrit value, and the hematocrit value and the amount of the substance to be measured may be determined with a high degree of accuracy.

This application claims priority on the basis of JP 2016-160756 A filed on August 18, 2016.

Industrial Applicability

Since the measurement method of the substance to be measured according to the present invention may highly reliably detect the substance to be measured, this is useful for examining diseases, for example.

Reference Signs List

| 10 | Measurement chip |
|----|----|
| 20 | Prism |
| 21 | Incident surface |
| 22 | Film-depositing surface |
| 23 | Emission surface |
| 30 | Reflecting unit |
| 30 | Metal film |
| 40 | Flow path lid |
| 41 | Flow path |
| 50 | Liquid chip |
| 100 | SPFS device |
| 110 | Excitation light emitting unit |
| 111 | First light source unit |
| 112 | Angle adjusting mechanism |
| 113 | First light source control unit |
| 120 | Signal detecting unit |
| 121 | Light receiving optical system unit |
| 122 | Position switching mechanism |
| 123 | First lens |
| 124 | Optical filter |
| 125 | Second lens |
| 126 | First light receiving sensor |
| 127 | First sensor control unit |
| 130 | Liquid sending unit |
| 131 | Pipette |
| 132 | Syringe pump |
| 133 | Nozzle unit |
| 134 | Pipette chip |
| 135 | Pipette control unit |

140    Transporting unit
141    Transporting stage
142    Chip holder
150    Light emitting unit
151    Second light source unit
152    Second light source control unit
160    Light detecting unit
161    Second light receiving sensor
162    Second sensor control unit
170    Control processing unit
$\alpha$      Excitation light (fourth light)
$\beta$      Fluorescence
$\gamma$      Plasmon scattered light
$\delta_1$     First light
$\delta'$     Reflected light of first light in measurement chip
$\delta_2$     Second light
$\delta_3$     Third light

**Claims**

1. A measurement method for measuring an amount of a substance to be measured in a specimen containing blood using a measurement chip (10) including an accommodating unit for accommodating liquid, the method comprising:

   a step of dividing the specimen into a first specimen and a second specimen;
   a step of hemolyzing the blood in the second specimen;
   a step of introducing the first specimen containing the substance to be measured into the accommodating unit ;
   a step of acquiring a measurement value indicating the amount of the substance to be measured in the first specimen in a state in which the blood is not hemolyzed;
   a step of introducing the second specimen into the accommodating unit;
   a step of shining first light ($\delta_1$) including light of a wavelength absorbed by red blood cells onto the second specimen in a state in which the blood is hemolyzed;
   a step of detecting second light ($\delta_2$) acquired when first light ($\delta_1$) passes through the second specimen in the accommodating unit in a state in which the second specimen is in a state in which the blood is hemolyzed ;
   a step of determining a hematocrit value of the specimen on the basis of a detection result of the second light ($\delta_2$); and
   a step of correcting the measurement value on the basis of the hematocrit value.

2. The measurement method according to claim 1, further comprising:
   a step of introducing a reference liquid transparent to the first light ($\delta_1$) in the accommodating unit; and a step of detecting third light ($\delta_3$) acquired when the first light ($\delta_1$) passes through the reference liquid in the accommodating unit ;
   wherein, at the step of determining the hematocrit value, the hematocrit value determined on the basis of the detection result of the second light ($\delta_2$) is corrected on the basis of a detection result of the third light ($\delta_3$).

3. The measurement method according to claim 1 or 2,
   wherein the accommodating unit is a flow path (41) through which liquid flows.

4. The measurement method according to any one of claims 1 to 3,
   wherein the step of detecting the second light ($\delta_2$) is performed after the step of acquiring the measurement value.

5. The measurement method according to claim 4,
   wherein the step of hemolyzing the blood in the second specimen is performed after the step of acquiring the measurement value.

6. The measurement method according to claim 5,
   wherein the step of dividing the specimen into the first specimen and the second specimen is performed within 10 minutes before the step of introducing the first specimen in the accommodating unit.

7. The measurement method according to any one of claims 1 to 6,
wherein the measurement chip (10) includes a prism (20), a metal film (30) arranged on the prism (20), and the accommodating unit arranged on the metal film (30), and
at the step of acquiring the measurement value, in a state in which the substance to be measured contained in the first specimen is immobilized on the metal film (30) and the first specimen and the second specimen are not present, a signal indicating the amount of the substance to be measured, generated when fourth light ($\alpha$) is applied to the metal film (30) through the prism (20) at an incident angle at which surface plasmon resonance occurs, is detected, and the measurement value is acquired.

8. The measurement method according to claim 7,
wherein the signal is fluorescence ($\beta$) emitted from a fluorescent substance which labels the substance to be measured.

**Patentansprüche**

1. Messverfahren zum Messen einer Menge einer zu messenden Substanz in einer Blut enthaltenden Probe unter Verwendung eines Mess-Chips (10), der eine Aufnahmeeinheit zum Aufnehmen von Flüssigkeit umfasst, wobei das Verfahren umfasst:

    einen Schritt des Aufteilens der Probe in eine erste Probe und eine zweite Probe;
    einen Schritt der Hämolyse des Blutes in der zweiten Probe;
    einen Schritt des Einführens der ersten Probe, die die zu messende Substanz enthält, in die Aufnahmeeinheit;
    einen Schritt der Erfassung eines Messwertes, der die Menge der zu messenden Substanz in der ersten Probe in einem Zustand anzeigt, in dem das Blut nicht hämolysiert ist;
    einen Schritt des Einführens der zweiten Probe in die Aufnahmeeinheit;
    einen Schritt des Einstrahlens von erstem Licht ($\delta_1$), das Licht mit einer Wellenlänge, die von roten Blutkörperchen absorbiert wird, umfasst, auf die zweite Probe in einem Zustand, in dem das Blut hämolysiert ist;
    einen Schritt des Detektierens von zweitem Licht ($\delta_2$), das erfasst wird, wenn erstes Licht ($\delta_1$) durch die zweite Probe in der Aufnahmeeinheit in einem Zustand, in dem die zweite Probe in einem Zustand ist, in dem das Blut hämolysiert ist, hindurchtritt;
    einen Schritt zum Bestimmen eines Hämatokritwertes der Probe auf der Grundlage eines Detektionsergebnisses des zweiten Lichts ($\delta_2$); und
    einen Schritt zur Korrektur des Messwertes auf der Grundlage des Hämatokritwertes.

2. Messverfahren nach Anspruch 1, weiter umfassend:

    einen Schritt des Einführens einer Referenzflüssigkeit, die für das erste Licht ($\delta_1$) transparent ist, in die Aufnahmeeinheit; und
    einen Schritt zur Detektion von drittem Licht ($\delta_3$), das erfasst wird, wenn das erste Licht ($\delta_1$) durch die Referenzflüssigkeit in der Aufnahmeeinheit hindurchtritt;
    wobei bei dem Schritt des Bestimmens des Hämatokritwertes der Hämatokritwert, der auf der Grundlage des Detektionsergebnisses des zweiten Lichts ($\delta_2$) bestimmt wurde, auf der Grundlage eines Detektionsergebnisses des dritten Lichts ($\delta_3$) korrigiert wird.

3. Messverfahren nach Anspruch 1 oder 2,
wobei die Aufnahmeeinheit ein Strömungsweg (41) ist, durch den Flüssigkeit fließt.

4. Messverfahren nach einem der Ansprüche 1 bis 3,
wobei der Schritt der Detektion des zweiten Lichts ($\delta_2$) nach dem Schritt der Erfassung des Messwertes durchgeführt wird.

5. Messverfahren nach Anspruch 4,
wobei der Schritt der Hämolyse des Blutes in der zweiten Probe nach dem Schritt der Erfassung des Messwertes durchgeführt wird.

6. Messverfahren nach Anspruch 5,
wobei der Schritt des Aufteilens der Probe in die erste Probe und die zweite Probe innerhalb von 10 Minuten vor dem Schritt des Einführens der ersten Probe in die Aufnahmeeinheit durchgeführt wird.

7.  Messverfahren nach einem der Ansprüche 1 bis 6,
    wobei der Mess-Chip (10) ein Prisma (20), einen auf dem Prisma (20) angeordneten Metallfilm (30) und die auf dem Metallfilm (30) angeordnete Aufnahmeeinheit umfasst, und
    beim Schritt der Erfassung des Messwertes in einem Zustand, in dem die in der ersten Probe enthaltene zu messende Substanz auf dem Metallfilm (30) immobilisiert ist und die erste Probe und die zweite Probe nicht vorhanden sind, ein Signal, das die Menge der zu messenden Substanz anzeigt, das erzeugt wird, wenn viertes Licht ($\alpha$) durch das Prisma (20) unter einem Einfallswinkel, bei dem Oberflächenplasmonenresonanz auftritt, auf den Metallfilm (30) gerichtet wird, detektiert wird und der Messwert erfasst wird.

8.  Messverfahren nach Anspruch 7,
    wobei das Signal Fluoreszenz ($\beta$) ist, die von einer fluoreszierenden Substanz emittiert wird, die die zu messende Substanz markiert.

## Revendications

1.  Procédé de mesure pour mesurer une quantité d'une substance à mesurer dans un spécimen contenant du sang à l'aide d'une puce de mesure (10) comportant une unité de logement pour recevoir un liquide, le procédé comprenant :

    une étape de division du spécimen en un premier spécimen et un deuxième spécimen ;
    une étape d'hémolyse du sang dans le deuxième spécimen ;
    une étape d'introduction du premier spécimen contenant la substance à mesurer dans l'unité de logement ;
    une étape d'acquisition d'une valeur de mesure indiquant la quantité de la substance à mesurer dans le premier spécimen dans un état dans lequel le sang n'est pas hémolysé ;
    une étape d'introduction du deuxième spécimen dans l'unité de logement ;
    une étape de projection d'une première lumière ($\delta_1$) comportant une lumière d'une longueur d'onde absorbée par les globules rouges sur le deuxième spécimen dans un état dans lequel le sang est hémolysé ;
    une étape de détection de la deuxième lumière ($\delta_2$) acquise lorsque la première lumière ($\delta_1$) traverse le deuxième spécimen dans l'unité de logement dans un état dans lequel le deuxième spécimen est dans un état dans lequel le sang est hémolysé ;
    une étape de détermination d'une valeur d'hématocrite du spécimen sur la base d'un résultat de détection de la deuxième lumière ($\delta_2$) ; et
    une étape de correction de la valeur de mesure sur la base de la valeur d'hématocrite.

2.  Procédé de mesure selon la revendication 1, comprenant en outre :

    une étape d'introduction d'un liquide de référence transparent à la première lumière ($\delta_1$) dans l'unité de logement ; et
    une étape de détection de troisième lumière ($\delta_3$) acquise lorsque la première lumière ($\delta_1$) traverse le liquide de référence dans l'unité de logement ;
    où, à l'étape de détermination de la valeur d'hématocrite, la valeur d'hématocrite déterminée sur la base du résultat de détection de la deuxième lumière ($\delta_2$) est corrigée sur la base d'un résultat de détection de la troisième lumière ($\delta_3$).

3.  Procédé de mesure selon la revendication 1 ou 2,
    dans lequel l'unité de logement est un chemin d'écoulement (41) à travers lequel s'écoule un liquide.

4.  Procédé de mesure selon l'une quelconque des revendications 1 à 3,
    dans lequel l'étape de détection de la deuxième lumière ($\delta_2$) est effectuée après l'étape d'acquisition de la valeur de mesure.

5.  Procédé de mesure selon la revendication 4,
    dans lequel l'étape d'hémolyse du sang dans le deuxième spécimen est effectuée après l'étape d'acquisition de la valeur de mesure.

**6.** Procédé de mesure selon la revendication 5,
dans lequel l'étape de division du spécimen en le premier spécimen et en le deuxième spécimen est effectuée dans les 10 minutes qui précédent l'étape d'introduction du premier spécimen dans l'unité de logement.

**7.** Procédé de mesure selon l'une quelconque des revendications 1 à 6, dans lequel la puce de mesure (10) comporte un prisme (20), un film métallique (30) agencé sur le prisme (20), et l'unité de logement agencée sur le film métallique (30), et
à l'étape d'acquisition de la valeur de mesure, dans un état dans lequel la substance à mesurer contenue dans le premier spécimen est immobilisée sur le film métallique (30) et le premier spécimen et le deuxième spécimen ne sont pas présents, un signal indiquant la quantité de la substance à mesurer généré lorsque la quatrième lumière ($\alpha$) est appliquée au film métallique (30) à travers le prisme (20) à un angle incident auquel la résonance plasmonique de surface se produit, est détecté ; et la valeur de mesure est acquise.

**8.** Procédé de mesure selon la revendication 7, dans lequel le signal est une fluorescence ($\beta$) émise par une substance fluorescente qui marque la substance à mesurer.

# FIG. 1

START

PREPARATION OF
MEASUREMENT — S110

DISPENSING AND
DILUTION OF SPECIMEN — S111

SET INCIDENT ANGLE
TO ENHANCEMENT ANGLE — S112

MEASUREMENT OF
OPTICAL BLANK VALUE — S113

PRIMARY REACTION — S114

SECONDARY REACTION — S115

MEASUREMENT OF
FLUORESCENCE VALUE — S116

HEMOLYSIS AND DILUTION — S117

ACQUIRE HEMATOCRIT
RELATED VALUE — S118

DETERMINE
HEMATOCRIT VALUE — S119

CORRECT
MEASUREMENT VALUE — S120

END

# FIG. 2

```
┌─────────────────────────┐
│   MEASUREMENT OF        │
│  OPTICAL BLANK VALUE    │
└─────────────────────────┘
            │
┌─────────────────────────┐  S1131
│   MEASUREMENT OF        │
│   FIRST BLANK VALUE     │
└─────────────────────────┘
            │
┌─────────────────────────┐  S1132
│   MEASUREMENT OF        │
│  SECOND BLANK VALUE     │
└─────────────────────────┘
            │
      ┌───────────┐
      │  RETURN   │
      └───────────┘
```

FIG. 3

*FIG. 4A*

*FIG. 4B*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015129615 A
- EP 2963418 A
- JP 2013036959 A
- JP 2016160756 A